# EUROPEAN PATENT SPECIFICATION

(11) **EP 1 305 010 B1**
(45) Date of publication and mention of the grant of the patent: **31.03.2004**
(21) Application number: 01976055.2
(22) Date of filing: 02.08.2001
(51) Int. Cl.: A61K 9/20, A61P 31/10

(54) **DOSAGE FORMS FOR TREATMENT OF ORAL MYCOSES**
DARREICHUNGSFORMEN ZUR BEHANDLUNG VON ORALEN MYKOSEN
FORMES POSOLOGIQUES POUR LE TRAITEMENT DE MYCOSES ORALES

(30) Priority: 03.08.2000 DE 10038571
(43) Date of publication of application: 02.05.2003
(73) Proprietor: Abbott GmbH & Co. KG, 65205 Wiesbaden (DE)
(72) Inventor: ROSENBERG, Jörg, 67158 Ellerstadt (DE); BERNDL, Gunther, 67273 Herxheim (DE); NEUMANN, Jörg, 35274 Kirchhain (DE); BREITENBACH, Jörg, 68199 Mannheim (DE)
(74) Representative: Riedl, Peter, Dr.
(86) International application number: PCT/EP2001/008978
(87) International publication number: WO 2002/011694

(56) References cited:
- EP-A- 0 240 906
- EP-A- 0 250 187
- EP-A- 0 864 326
- WO-A-93/18757
- WO-A-97/44014
- DE-A- 19 539 359
- US-A- 5 431 915
- US-A- 5 578 315

## Description

The present invention relates to the use of a solid dosage form comprising itraconazole molecularly dispersed in a pharmaceutically acceptable matrix, which is obtained by a melt-extrusion process, for the manufacture of a medicament for the treatment of oral mycoses, especially mycosis caused by Candida albicans, by application in the oral cavity.

Since administration of drugs in all regions from the neck up avoids first-pass metabolism, administration in the oral cavity seems to be a very efficacious way to deliver systemic drugs.

This is especially important in the case of pharmaceutically active ingredients which show poor bioavailability due to first pass metabolism and/or poor water-solubility.

In the treatment of oral mykoses it is particularly advantageous to provide for relatively high local concentrations of the active ingredient in the oral cavity.

Itraconazol, (±)-cis-4-[4-[4-[4-[[2-(2,4-dichlorophenyl)-2-(1H-1,2,4-triazol-1-yl-methyl)-1,3-dioxolan-4-yl]methoxy]phenyl]-1-piperazinyl]phenyl]-2,4-dihydro-2-(1-methylpropyl)-3H-1,2,4-triaz ol-3-one, and its pharmaceutically acceptable salts, is known as an effective active ingredient for oral, parenteral and topic treatment of various types of mykoses. Predominantly, itraconazol is administered orally because of its tendency of extensive tissue distribution.

However, since itraconazole is almost insoluble in water (less than 1 µg/ml), bioavailability is a major problem.

Many attempts have been made to improve the bioavailability of almost insoluble drug compounds. Among them, solid dispersions of drug and hydrophilic polymers have been suggested to enhance the solubility of the drug.

WO 97/44014 discloses particles, comprising formulations of itraconazole and water-soluble polymers, said formulations being obtained by melt-extrusion, preferably using hydroxypropyl methylcelluloses as water-soluble polymers. The oral dosage forms disclosed in that document show a remarkably lower food effect.

According to WO 95/31178 mucoadhesive emulsion formulations , comprising itraconazole and cyclodextrins, are useful in the treatment of vaginal infections.

The object of the present invention is to provide formulations and dosage forms for application in the oral cavity for the treatment of mykoses, especially mykoses of the oral cavity.

Formulations according to the present invention comprise an antimycotic active ingredient in the form of solid dispersions of the active ingredient in a pharmaceutically acceptable matrix, particularly molecular dispersions of the active ingredient in the polymer.

The active ingredient is the above-identified itraconazole.

One preferred embodiment of the invention relates to lozenges.

Another preferred embodiment of the invention relates to solid dosage forms comprising mucoadhesive polymers, preferably tablets for sublingual or buccal application. Tablets for gingival or palatal application are also within the scope of the invention.

According to the present invention the active ingredient is homogeneously dispersed in a pharmaceutically acceptable matrix. Preferably, the solid dispersion is in the form of a molecular dispersion of the active ingredient, i.e. a so-called "solid solution". The term "solid solution" is familiar to the skilled person.

The pharmaceutically acceptable matrix is based on polymers or low-molecular excipients normally used as fillers for tabletting such as for instance sugars or sugar alcohols as matrix building components.

Suitable polymers are selected from the group consisting of:
Cellulose derivatives, e.g. alkylcelluloses, hydroxyalkylcelluloses, hydroxyalkyl alkylcelluloses.
Acrylic polymers of the Eudragit® type like copolymers based on methacrylic acid and methycrylic acid methyl ester
Homo- and copolymers of N-vinylpyrrolidone with Fikentscher K values in the range of from 17 to 100, vinylacetate being a preferred comonomer, e.g. a copolymer obtained from 60 % b.w. of n-vinylpyrrolidone and 40 % b.w. of vinyl acetate
Polyethylene glycols with molecular weights in the range of from 6000 to 100.000 Dalton, polyoxyethylene polyoxypropylene block copolymers

Suitable low-molecular weight matrix components are selected from the group consisting of sugars and sugar alcohols, for example sorbitol, xylitol, maltitol, erythritol, mannitol, isomalt and the like.

In the case of formulations for lozenges sugar alcohols are preferred matrix components.

The amount of matrix building components used in the formulations may range from 5 to 90 % b.w., preferably 10 to 70 % b.w., more preferably 10 to 50 % b.w..

Notwithstanding the fact that some of the aforementioned matrix building polymers show mucoadhesive properties, such polymers are only used optionally in formulations for lozenges. However, formulations or finished dosage forms for buccal, sublingual, gingival or palatinal application preferably comprise such mucoadhesive polymers, optionally in combination with other polymers. Such mucoadhesive polymers are selected form the group consisting of:
Acrylic copolymers of the Eudragit© type
Crosslinked polyacrylics (CTFA name: Carbomer)
Sodium carboxymethylcellulose
Tragant gum
Poly(methyl)vinylether-co- maleic acid anhydride
Alkylcelluloses, e.g. methylcellulose
Alginates like sodium alginate
Polyvinylpyrrolidone

According to one embodiment of the invention the mucoadhesive polymer is incorporated in the melt formulation.

According to another embodiment of the invention the solid dispersions of the active ingredient obtained by melt formulation are mixed with on or more mucoadhesive polymers and subsequently processed to finished dosage forms (tablets). For example, 10 to 70 % b.w. of a solid dispersion of the active ingredient in a pharmaceutically acceptable matrix as outlined above can be mixed with 30 to 90 % b.w. of mucoadhesive polymers.

In addition the matrix formulations or finished dosage forms may contain conventional pharmaceutical ancillary substances, for example extenders such as silicats or diatomaceous earth, mould releasers such as stearic acid or salts thereof, wettings agents, preservatives, disintegrants, absorbants, colorants, and the like (cf., for example H. Sucker et al. Pharmazeutische Technologie, Thieme Verlag, Stuttgart 1978). The ancillary substances must be thermally stable at the temperature used in the process for manufacture used here.

Preferred ancillary substances are flavourings and artificial sweeteners for masking the sometimes unpleasant taste of the drug compounds. Suitable artificial sweeteners are for instance sodium saccharinate, aspartame, neohesperidine or acesulfame, preferably acesulfame or mixtures comprising acesulfame and aspartame. These sweeteners are used in amounts of from 0.05 to 1.0 % b.w., preferably 0.2 to 0.5 to 0.5 b.w.. Another preferred class of sweeteners are sugar alcohols, preferably xylitol, maltitol or isomalt. In case sugar alcohols are used as matrix components additional sweeteners normally are not needed. Sugar alcohols can be used in amounts of from 2 to 60 % b.w., preferably 5 to 40 % b.w..

The formulations and finished dosage forms of the present invention are obtained by a melt-extrusion process. A preferred apparatus for such process is an extruder equipped with one or more screws, preferably a twin screw extruder. The mixtures comprising all the components of the pharmaceutical formulations can be processed at temperatures in the range of from 50 to 180°C, preferably 80 to 160°C. Preferably the process is carried out in the absence of solvents, e.g. water or organic solvents.

In addition, small amounts of crosslinked polyvinylpyrrolidone (Kollidon® CL) can be used as taste masking agent.

The molten pharmaceutical mixtures are extruded and the still thermoplastic mass is subsequently shaped. Shaping can take place e.g. by hot cutting the extruded strands to give granules or pellets which can be pressed to tablets in a conventional way.

A preferred method for shaping is a calendering process as described for instance in EP-A 240 906 which comprises that the still deformable extrudate is fed between the surfaces of two counter-rotating molding rolls, the surfaces of said rolls having opposed depressions, whereby, separate tablets having the shape of such depressions are formed. The calender and molding rolls useful for the present invention can be cooled or heated per se and the optimum surface temperature of the rolls for the relevant processing step can be adjusted in this way.

The invention also relates to specifically shaped dosage forms for those formulations comprising mucoadhesive polymers.

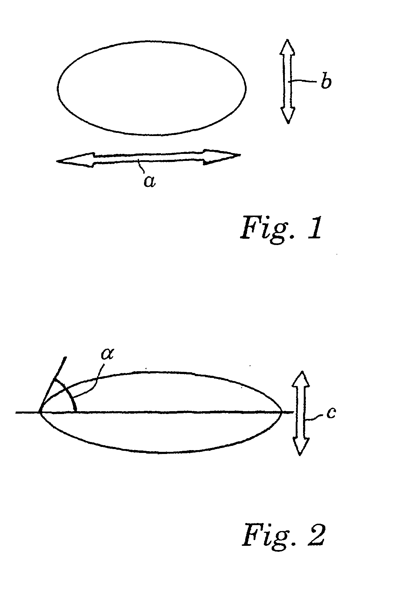

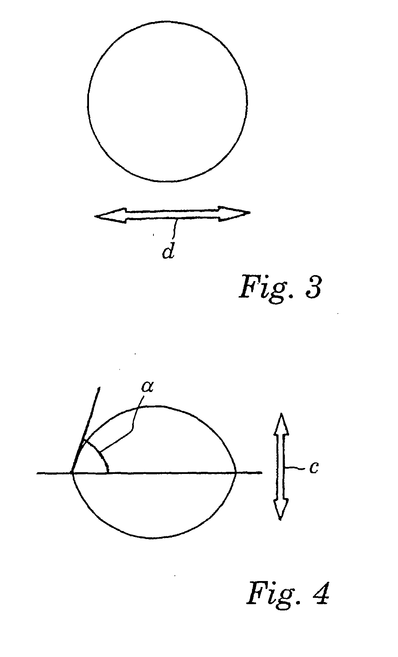

Preferred dosage forms are lenticular or semi-lenticular tablets which can be round or oval and with an angle á (see Fig.)between the cross-sectional plane of the tablet and the convex tablet body (tangential area)of less than 90°. Fig. 1 and 2 show such an oval lenticular tablet with a tablet length (a), tablet width (b) and thickness (c). Fig. 3 and 4 show a round lenticular tablet with a diameter (d) and a thickness (c).

For instance, oval lozenges can have a length of from 10 to 20 mm, a width of from 6 to 12 mmm and and a thickness of from 3 to 12 mm. Round lozenges can have a diameter of from 5 to 14 mm and a thickness of from 3 to 10 mm.

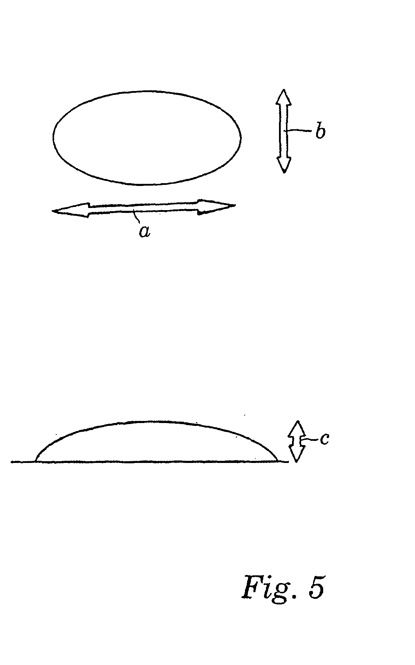

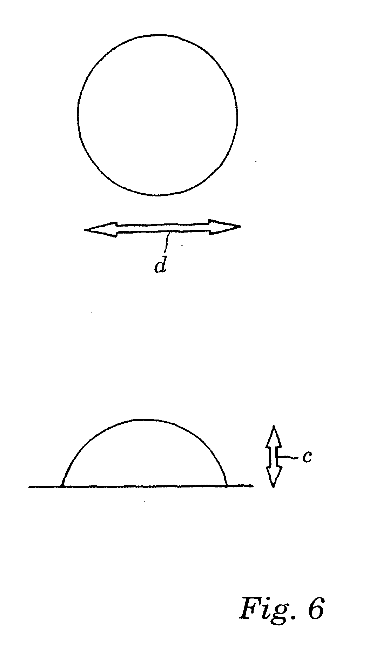

In the case of semi-lenticular tablets the lower half of the tablet is essentially planar. Fig. 5 shows a round semi-lenticular tablet, Fig. 6 an oval semi-lenticular tablet. Such tablet forms are especially well suited for buccal, gingival, sublingual or palatal application, since they cause little irritation when positioned in the oral cavity. Also, in view of the low tablet weight generally accepted for buccal forms (up to 200 mg per tablet) the ratio of surface to tablet volume is particularly advantageous because of the large surface. Such tablets can have a length of from 3 to 10 mm, a width of from 2 to 6 mmm, a thickness of from 1.5 to 5 mm (oval forms) or a diameter of from 3 to 10 mm and a thickness of from 1.5 to 5 mm (round forms). Round semi-lenticular tablets are preferred.

Such dosage forms can be manufactured using a calendering process as described above. In the case of semi-lenticular tablets only one of the calender rolls is having depressions, whereas the other roll is planar.

Another method for manufacturing semi-lenticular tablets is by shaping the melt with the aid of a rotating perforated roll into drops which are subsequently solidified by cooling.

The dosage forms obtained according to the present invention are particularly useful in the treatment of oral mykoses.

Surprisingly, the solid solutions according to the present invention deliver the active ingredient without substantial recristallization in an aqueous environment like the oral cavity, thus achieving sufficient plasma levels.

Therefore, the dosage forms according to the invention are useful in the treatment of diseases of the oral cavity by delivering high local concentrations.

### Examples

### General Method

Tablets were produced starting from molten mixtures of the components and extruding said mixtures using a twin screw extruder (Leistritz Micro 18). The still thermoplastic extrudate was calendered as described in EP-A 240 906 to give oval lozenges of 17,4 mm length, 8.5 mm width and 4.7 mm thickness with a mean tablet weight of 450 mg.

The dissolution rates were determined according to the USP-paddle model at 50 rpm. 37°C, no change pH 1.0 (0.5 % SDS).

The formation of solid solutions was determined by DSC (Differential Scanning Calorimetry) measurements using a Mettler TA 4000 System.

### Example 1

| | |
|---|---|
| Itraconazol | 20 % b.w. |
| Hydroxypropylcellulose | 80 % b.w. |
| Melt temperature: | 133°C |
| Dissolution: 95 % after 8 h | |

### Example 2

| | |
|---|---|
| Itraconazol | 20 % b.w. |
| Hydroxypropylcellulose | 70 % b.w. |
| Hydroxypropylmethylcellulose | 10 % b.w. |
| Melt temperature: | 135°C |
| Dissolution: 77 % after 8 h | |

### Example 3

| | |
|---|---|
| Itraconazol | 20 % b.w. |
| N-vinylpyyrolidone vinylacetate | |
| Copolymer (VP/Vac 60/40) | 60 % b.w. |
| Hydroxypropylcellulose | 10 % b.w. |
| Melt temperature: | 152°C |
| Dissolution: 93 % after 8 h | |

## Claims

1. The use of a solid dosage form comprising itraconazole molecularly dispersed in a pharmaceutically acceptable matrix, which is obtained by a melt-extrusion process for the manufacture of a medicament for the treatment of oral mycoses by application in the oral cavity.

2. The use according to claim 1, wherein the dosage form is in the form of a lozenge.

3. The use according to claims 1 or 2, wherein the dosage form comprises one or more sugar alcohols as matrix components.

4. The use according to any of the claims 1 to 3, wherein the dosage form comprises mucoadhesive polymers.

5. The use according to claim 4, wherein the mucoadhesive polymer is a crosslinked polyacrylic acid.

6. The use according to claim 4, wherein the mucoadhesive polymer is a poly(math)acrylate.

7. The use according to any of the claims 4 to 6, wherein the dosage form is in the form of a tablet for buccal, sublingual, gingival or palatinal application

8. The use according to claim 7, wherein the dosage form consists of lenticular or semi-lenticular shaped tablets.

## Patentansprüche

1. Verwendung einer festen Dosierungsform, die Itraconazol in molekularer Dispersion in einer pharmazeutisch verträglichen Matrix enthält, welche durch ein Schmelzextrusionsverfahren erhalten ist, zur Herstellung eines Arzneimittels zur Behandlung oraler Mykosen durch Anwendung in der Mundhöhle.

2. Verwendung nach Anspruch 1, wobei die Dosierungsform in Form einer Lutschtablette vorliegt.

3. Verwendung nach Anspruch 1 oder 2, wobei die Dosierungsform einen oder mehrere Zuckeralkohole als Matrixkomponenten umfasst.

4. Verwendung nach einem der Ansprüche 1 bis 3, wobei die Dosierungsform mukoadhäsive Polymere umfasst.

5. Verwendung nach Anspruch 4, wobei es sich bei dem mukoadhäsiven Polymer um vernetzte Polyacrylsäure handelt.

6. Verwendung nach Anspruch 4, wobei es sich bei dem mukoadhäsiven Polymer um ein Poly(meth)acrylat handelt.

7. Verwendung nach einem der Ansprüche 4 bis 6, wobei die Dosierungsform in Form einer Tablette zur bukkalen, sublingualen, gingivalen oder palatalen Anwendung vorliegt.

8. Verwendung nach Anspruch 7, wobei die Dosierungsform aus linsenförmigen oder halblinsenförmigen Tabletten besteht.

## Revendications

1. Utilisation d'une forme posologique solide comprenant de l'itraconazole sous forme de dispersion moléculaire dans une matrice pharmaceutiquement acceptable, qui est obtenue par un procédé d'extrusion en fusion, pour la fabrication d'un médicament pour le traitement des mycoses orales par application dans la cavité orale.

2. Utilisation selon la revendication 1, dans laquelle la forme posologique se présente sous forme d'une pastille.

3. Utilisation selon la revendication 1 ou 2, dans laquelle la forme posologique comprend un ou plusieurs alcools de sucre en tant que composants de la matrice.

4. Utilisation selon l'une quelconque des revendications 1 à 3, dans laquelle la forme posologique comprend des polymères mucoadhésifs.

5. Utilisation selon la revendication 4, dans laquelle le polymère mucoadhésif est un acide polyacrylique réticulé.

6. Utilisation selon la revendication 4, dans laquelle le polymère mucoadhésif est un poly(méth)acrylate.

7. Utilisation selon l'une quelconque des revendications 4 à 6, dans laquelle la forme posologique se présente sous forme d'un comprimé pour application buccale, sublinguale, gingivale ou palatinale.

8. Utilisation selon la revendication 7, dans laquelle la forme posologique consiste en des comprimés de forme lenticulaire ou semi-lenticulaire.
